# EUROPEAN PATENT APPLICATION

(11) **EP 1 813 307 A1**
(43) Date of publication of application: **01.08.2007**
(21) Application number: 07100083.0
(22) Date of filing: 04.01.2007
(51) Int. Cl.: A61N 5/06

(54) **Phototherapy light with fresnel lens for infant care apparatus**

(30) Priority: 06.01.2006 US 327554
(71) Applicant: The General Electric Company, Schenectady NY 12345 (US)
(72) Inventor: Hodge, Colin G., Ellicott City, MD 21042 (US)
(74) Representative: Bedford, Grant Richard

(57) **Abstract**

A phototherapy light device (10) for directing light onto an infant. The phototherapy light device (10) has a base (12) containing an illumination source (32) and a gooseneck type of elongated member (22) that extends outwardly from the base (12) and has a conical shaped shade (62) at its outer end (24). An optical fiber (38) transmits the light from the illumination source (32) to the shade (62) through the optical fiber (38) where the light is passed through a plastic Fresnel lens (82) at the distal end (66) of the shade (62). By the use of the Fresnel lens (82) and the means of affixing the shade (62) onto the elongated member (22), the distal end of the phototherapy light device (10) is relatively light and which alleviates the problem of that distal end sagging downwardly to contact the infant undergoing treatment.

## Description

The present invention relates generally to a phototherapy light for treating infants and, more particularly, to a phototherapy light having an improved lens.

Hyperbilirubinemia is an affliction of newborn infants typified by an elevated level of a toxic molecule know as bilirubin in the infant's blood. Current medical therapy for such affliction is through the use of phototherapy where light radiation, generally within a certain wavelength band is directed upon the infant's skin.

There are a number of means or devices that are conventionally employed to direct the light upon the skin of the infant, one of which is to provide a bank of lights that are portable or can be built into an infant care apparatus and which direct the light radiation toward the infant positioned on an infant platform as a part of that apparatus.

Another means of providing phototherapy is through the use of a fiberoptic pad that is comprised of woven optical fibers such that the light radiation couples out of the optical fibers at the various bends that are formed in the optical fibers during the weaving process.

A still further means or device is by the use of a "gooseneck" phototherapy device that has a maneuverable, elongated neck that the caregiver can bend to the desired position to direct the light radiation upon the area of the infant where the light radiation is needed.

With the "gooseneck" type of phototherapy device, the distal end that is positioned so as to direct the light radiation upon the infant conventionally includes a lens that focuses the light radiation to most effectively impinge on the infant.

One difficulty, however, in the use of the device, is that the lens is comprised of a relatively heavy glass material and, since the distal end of the "gooseneck" can be suspended over the infant, the weight of the lens can cause drooping or sagging of the distal end and can result in the distal end of the "gooseneck" phototherapy device actually contacting the infant. Since the glass lens is normally at an elevated temperature, the contact with the infant is obviously not desirable since it can cause harm to the infant.

It would be advantageous to have a design and construction of a "gooseneck" type of phototherapy device that provides the necessary alleviation of the bilirubin condition but with a lighter distal end. It would be even further advantageous if the distal end of that phototherapy device were maintained at a relatively low temperature to reduce the potential harm to the infant if there is still an inadvertent contact with an infant.

Accordingly, one aspect of the present invention relates to a phototherapy light device that is usable in the treatment of bilirubinemia of an infant. With the device of the present invention, the device preferably has a base where the source of illumination is located for the light therapy. Extending from the base is an elongated "gooseneck" type of flexible member that terminates at a distal end. The elongated member encloses a light pipe or optical fiber system that transmits the light from the light source in the base to the distal end where the light can emanate to impinge upon the infant.

By means of the flexibility of the elongated member, the location of the distal end can be manipulated by the user to direct the light upon the desired area of the infant while the base remains in a stationary position.

At the distal end of the elongated member, there is a housing that has an opening for the distal end of the optical fiber to retain it in a desired location and the housing flares outwardly in a generally conical configuration to a widened end where there is located a Fresnel lens.

As such, the light emanating from the distal end of the optical fiber radiates somewhat outwardly in a diverging pattern to reach the inner surface of the Fresnel lens where the light is redirected outwardly from the housing in a generally collimated pattern. In the present invention, the inner surface of the Fresnel lens is ridged and which faces the distal end of the optical fiber while the outer surface that faces the infant is the smooth side of the Fresnel lens.

The effect of the Fresnel lens is to prevent the buildup of heat that can cause harm to the infant if there is contact therebetween. The Fresnel lens has a larger radius than the prior art glass lens. In addition, the Fresnel lens is preferable plastic so that the combination of the large area and plastic material allows the Fresnel lens to redirect the light without creating significant heat. Thus, the distal end of the phototherapy light device is not greatly heated and the hazard of contact with an infant by a heated surface is eliminated.

In addition, the Fresnel lens is lighter that the prior use of a glass lens and that lighter construction as well as the particular assembly of the housing to the elongated member also aids in alleviating the problem of the distal end of the phototherapy light device sagging downwardly and possible contacting the infant.

These and other features and advantages of the present invention will become more readily apparent during the following detailed description taken in conjunction with the drawings herein, in which:
Fig. 1 is a perspective view of a phototherapy light device constructed in accordance with an embodiment of the present invention;
Fig. 2 is a side cross sectional view of the elongated member used in an embodiment of the present invention;
Fig.3 is an exploded view illustrating certain components and their assembly in constructing the phototherapy light device of an embodiment of the present invention; and Fig. 4 is a perspective view of a wave ring used in an embodiment of the invention in the assembly of the phototherapy light device.

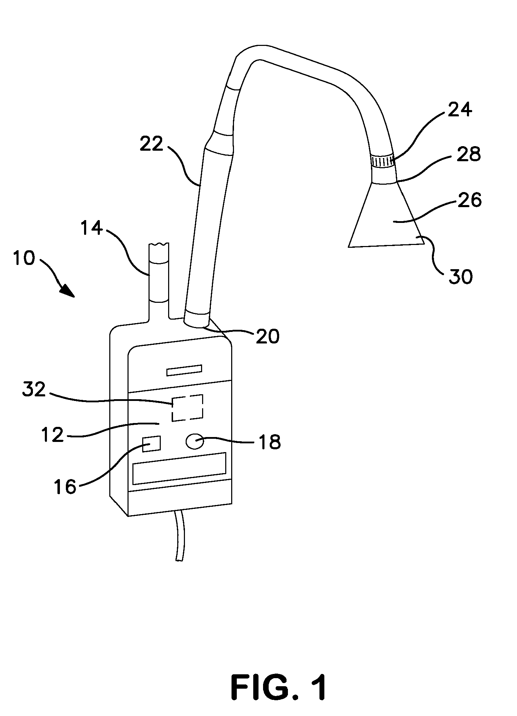

Referring now to Fig. 1, there is shown a perspective view of the phototherapy light device 10 constructed in accordance with an embodiment of the present invention. As can be seen, the phototherapy light device 10 includes a base 12 and, in the embodiment shown, the base 12 can be mounted to a stand 14 so that the phototherapy light device 10 can be moved to a location proximate to an infant for use in administering phototherapy. As an alternative, the base 12 can be affixed to an infant care apparatus, such as an incubator or infant warmer or may be physically incorporated into an infant care apparatus.

There can be suitable controls on the base 12, including a power switch 16 and an intensity control 18 to allow the user to set a desired intensity of the phototherapy light directed onto an infant. In the upper surface of the base 12 there is an opening 20 where an elongated member 22 is affixed to the base 12.

The elongated member 22 itself can be a flexible member such as is used in a gooseneck lamp so that the user can manipulate the elongated member 22 as desired so as to position the distal end 24 of the elongated member 22 in a particular location to direct the phototherapy light at a selected area of the infant. Affixed to the distal end 24 of the elongated member 22 is a shade assembly 26 that is generally conical shaped with a reduced diameter proximal end 28 and a larger diameter distal end 30.

Thus, overall, there is an illumination source 32 located in the base 12 and which provides the light that is emitted from the distal end 30 of the shade assembly 26 to impinge upon the infant. As is conventional, the illumination source 32 can be a light bulb, for example, of about 50 watts, that provides light at the preferred wavelength to alleviate the bilirubinemia.

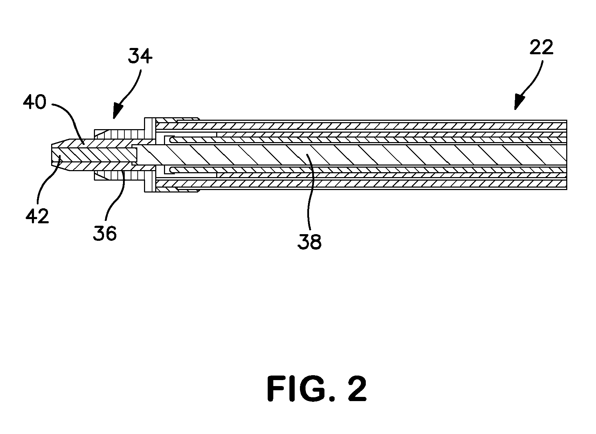

Turning now to Fig 2, taken along with Fig. 1, there is shown a side cross sectional view of a portion of the elongated member 22 and, in particular, illustrating the proximal end 34 of the elongated member 22. As can be seen, the proximal end 34 includes a circular flange 36 that is dimensioned so as to interfit into the opening 20 in the base 12 in order to affix the elongated member 22 to the base 12. Within the elongated member 22 there is an optical fiber 38 that runs the full length of the elongated member 22, that is, from the proximal end 34 to the distal end 24. The optical fiber 38 may be protected by a shield 40 that covers the full length of the optical fiber 38.

Accordingly, in conventional manner, the proximal end 34 of the elongated member 22 is affixed firmly within the opening 20 in the base 12 such that the proximal end 42 of the optical fiber 38 is optically located proximate to the illumination source 32 to enable the light from the illumination source 32 to enter the proximal end 42 of the optical fiber 38 so as to transmit that light to the distal end 24 of the elongated member 22.

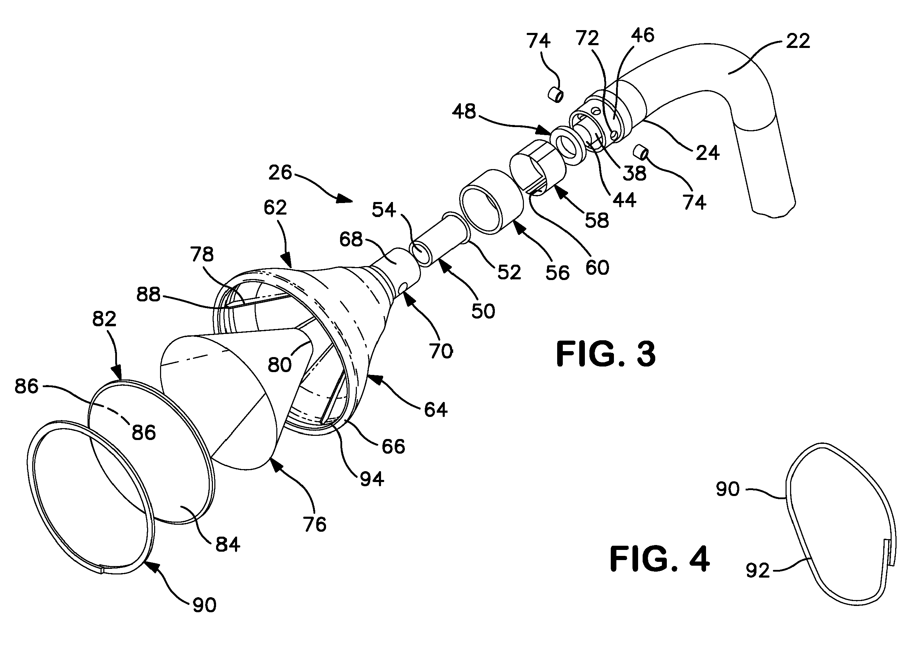

Turning now to Fig. 3, taken along with Figs. 1 and 2, there is shown an exploded view illustrating certain components and the assembly and affixation of the shade assembly 26 to the distal end 24 of the elongated member 22. As will be seen, the optical fiber 38 and the elongated member 22 are both affixed to the shade assembly 26 in a manner that facilitates the assembly during the manufacturing process. As such, therefore, the distal end 44 of the optical fiber 38 is positioned within a fitting 46 that is affixed at the distal end 24 of the elongated member 22. A foam washer 48 is slipped over the distal end 44 of the optical fiber 38 and an aperture sleeve 50 is also slipped over that distal end 44. In the process, as the sleeve 50 is placed onto the distal end 44, a inner flange 52 on the sleeve 50 contacts and pushes the foam washer 48 inwardly and which serves as an inner seal to seal between the optical fiber 38 and the internal diameter of the elongated member 22.

Thus the distal end 44 of the optical fiber 38 is encased in the aperture sleeve 50 that has an aperture 54 at its external end in order to allow the light to pass from the distal end 44 of the optical fiber 38 into the shade assembly 26 in a manner that will be later explained.

In addition, as can be seen, there is an elastomeric sleeve 56 comprises of a flexible material and a retention clip 58 that can be made of a steel material having a slit 60 along its length and the purpose of the elastomeric sleeve 56 and the retention clip 58 will be later explained, however at this point in the assembly, the elastomeric sleeve 56 and the retention clip 58 are assembled to the shade assembly 26 by begin slipped over the distal end 24 of the elongated member 22.

The coupling of the elongated member 22 to the sleeve assembly 26 is accomplished by affixing a shade 62 to the distal end 24 of the elongated member 22. The shade 62 has a generally conical shaped body 64 with a large diameter outer end 66 and a small, cylindrical inner end 68. In the assembly, therefore, the cylindrical inner end 68 of the shade 62 is interfitted over the aperture sleeve 52 and inside of the fitting 46. There are a plurality of apertures 70, generally two apertures, located 180 degrees apart, in the cylindrical inner end 68 of the shade 62 that align with apertures 72 in the fitting 46 such that pins 74 can be inserted through the respective, aligned apertures 70 and 72 to affix the shade 62 to the fitting 46, thereby, of course, affixing the shade 62 to the distal end 24 of the elongated member 22.

The retention clip 58 can then be slid distally along the elongated member 22 to a position overlying the pins 74 to retain the pins 74 in their position affixing the shade 62 to the fitting 46. The elastomeric sleeve 56 can then also be slipped distally along the elongated member 22 to a position overlying the retention clip 58 to provide a moisture seal over the retention clip 58 as well as other areas of the junction between the elongated member 22 and the shade assembly 26.

As such with the shade 58 to the elongated member 22, the remaining components of the shade assembly 26 can be assembled. Thus, a reflective cone 76 is inserted into the interior of the shade 62 and rests against ribs 78 formed on the interior surface of the shade 62. The inner end 80 of the reflective cone 76 fits around the aperture 54 in the aperture sleeve 50 to allow the light emanating from the distal end 44 of the optical fiber 38 to enter and be reflected off of the interior metallized surface of the reflective cone 76.

A Fresnel lens 82 is located at the distal end outer end 66 of the shade 62 and is preferably made of a plastic material for lightness. Since a Fresnel lens, by definition, has a flat side and a ridged side, in the present construction the flat side is the outer side 84, that is, facing toward the infant and the ridged side is the inner side 86 facing the optical fiber 38. By that orientation it can be assured that the outer side 84 can be easily cleaned by the users since it is flat without the ridges which are difficult to clean.

As can be seen, the Fresnel lens 82 rests against the outer ends 88 of the ribs 78 to hold the Fresnel lens 82 along its inner side 86 and a wave spring 90 affixes the Fresnel lens to the shade 62. The wave spring 90 is better shown in Fig. 4, taken along with Fig. 3 and used to hold the Fresnel lens 82 safely to the outer end 66 of the shade 62. Basically, the wave spring 90 is a circular ring of overlapping ends and which has waves or various sinusoidal indentations 92 formed therein. In assembling the Fresnel lens 82 to the outer end 66 of the shade 62, the Fresnel lens 82 is placed into a circular groove 94 formed in the outer end 66 and the wave spring 90 is compressed and sprung open in the annular grove 94 to retain the Fresnel lens 82 firmly in position.

As described and shown, therefore, it can be seen that the shade assembly 26 is affixed to the distal end 24 of the elongated member 22 is a positive manner and yet which does not add significant weight to the distal end 24 of the elongated member 22 so that the phototherapy light device can be held in its desired position over the infant and not sag toward the infant.

Therefore, with various embodiments of the present invention, the light originating from the illumination source 32 passes through the optical fiber 38 to the distal end 44 of the optical fiber 38 where it enters the housing shade 62 to impinge on the inner side 86 of the Fresnel lens 82. That ridged inner side 86 then passes the light to the smooth, outer side 84 of the Fresnel lens 82 to be directed toward the infant at the desired area.

By means of the "gooseneck" elongated member 22, the light can be directed by the user to the desired area of the infant. By the particular arrangement of affixing the shade assembly 26 to the elongated member 22, and the use of a light, plastic Fresnel lens, the distal end of the phototherapy light device 10 is sufficiently light as compared to the prior art glass lens unit, to allow the distal end of the phototherapy light device 10 to be safely positioned over an infant and reduce the possibility of sagging and potential touching of the infant.

Those skilled in the art will readily recognize numerous adaptations and modifications which can be made to the phototherapy light device of embodiments of the present invention which will result in an improved phototherapy system for an infant care apparatus, yet all of which will fall within the scope and spirit of the present invention as defined in the following claims. Accordingly, the invention is to be limited only by the following claims and their equivalents.

### PARTS LIST

| | |
|---|---|
| 10 | phototherapy light device |
| 12 | base |
| 14 | stand |
| 16 | power switch |
| 18 | intensity control |
| 20 | opening - in base |
| 22 | elongated member |
| 24 | distal end - of elongated member |
| 26 | shade assembly |
| 28 | proximal end - of shade assembly |
| 30 | distal end - of shade assembly |
| 32 | illumination source |
| 34 | proximal end - of elongated member |
| 36 | circular flange |
| 38 | optical fiber |
| 40 | shield |
| 42 | proximal end - of optical fiber |
| 44 | distal end - of optical fiber |
| 46 | fitting |
| 48 | foam washer |
| 50 | aperture sleeve |
| 52 | inner flange |
| 54 | aperture |
| 56 | elastomeric sleeve |
| 58 | retention clip |
| 60 | slit |
| 62 | shade |
| 64 | conical shaped body - of shade 62 |
| 66 | outer end - of shade |
| 68 | inner end - of shade |
| 70 | aperture - in inner end of shade |
| 72 | aperture - in fitting 46 |
| 74 | pin |
| 76 | reflective cone |
| 78 | rib |
| 80 | inner end - of reflective cone |
| 82 | Fresnellens |
| 84 | outer side - of Fresnel lens |
| 86 | inner side - of Fresnel lens |
| 88 | outer ends - of ribs |
| 90 | wave spring |
| 92 | sinusoidal indentations- in wave spring |
| 94 | circular groove |

## Claims

1. A phototherapy light device (10) for treating infants, the phototherapy light device (10) comprising a base (12), an elongated member (22) having an outer end (24) extending from the base (12), a shade (62) located at the outer end (24) of the elongated member (22), the shade (62) having a distal end (66) extending away from the elongated member (22), a source of light (32) to provide light radiating from the distal end (66) of the shade (62), the elongated member (22) being maneuverable so as to direct the light radiating from the distal end (66) of the shade (62) to impinge on an infant, and a Fresnel lens (82) located at the distal end (66) of the shade (62) wherein the light radiated from the distal end (62) of the shade (62) passes through the Fresnel lens (82).

2. The phototherapy light device (10) as defined in claim 1 wherein the source of light (32) comprises a bulb located in the base (12) and an optical fiber (38) system that transmits the light from the bulb to the distal end (66) of the shade (62).

3. The phototherapy light device (10) as defined in claim 1 or claim 2 wherein the Fresnel lens (82) has a smooth surface (84) facing outwardly toward an infant and a ridged surface (86) facing inwardly toward the elongated member (22).

4. The phototherapy light device (10) as defined in any preceding claim wherein the shade (62) is affixed to the elongated member (22) by means of pins (74).

5. The phototherapy light device (10) as defined in any preceding claim wherein the Fresnel lens (62) is affixed to the distal end (66) of said shade (62) by means of a wave spring (90).

6. A infant care apparatus, said infant care apparatus comprising a structure having a phototherapy light device (10) affixed to the structure, the phototherapy light device (10) comprising a base (12), an elongated member (22) having an outer end (24) extending from the base (12), a shade (62) located at the outer end (24) of the elongated member (22), the (62) shade having a distal end (66), a source of light (32) to provide light radiating from the distal end (66) of the shade (62), the elongated member (22) being maneuverable so as to direct the light radiating from the distal end (66) of the shade (62) to impinge on an infant when resting on the infant care apparatus, and a Fresnel lens (82) located at the distal end (66) of the shade (62) wherein the light radiated from said distal end (66) of said shade (62) passes through the Fresnel lens (82).

7. The infant care apparatus as defined in claim 6 wherein the source of light (32) comprises a bulb located in the base (12) and an optical fiber system (38) that transmits the light from the bulb to the distal end (66) of the shade (62).

8. The infant care apparatus as defined in claim 6 or claim 7 wherein the Fresnel lens (82) has a smooth surface (84) facing outwardly toward an infant and a ridged surface (86) facing inwardly toward the elongated member (22).

9. A method of providing phototherapy light to an infant comprising the steps of:
providing a base (12) having an elongated member (22) extending therefrom and having a shade (62) located at an outer end (24) of the elongated member (22), the shade (62) having a distal end (66) adapted to be positionable at a desired location with respect to an infant,
causing light to be emitted from the distal end (66) of the shade (62),
providing a Fresnel lens (82) at the distal end (66) of the shade (62) such that the light emitted from the shade (62) passes through the Fresnel lens (82).

10. The method as defined in claim 9 wherein the step of providing a Fresnel lens (82) comprises providing a Fresnel lens (82) that is oriented to have its ridged side (86) facing the elongated member and its smooth side (84) facing an infant.
